# EUROPEAN PATENT SPECIFICATION

(11) **EP 1 392 313 B1**
(45) Date of publication and mention of the grant of the patent: **18.04.2007**
(21) Application number: 02743024.8
(22) Date of filing: 15.05.2002
(51) Int. Cl.: A61K 31/506, A61P 35/00, A61P 37/06, A61K 31/675

(54) **COMBINATION COMPRISING N- 5- 4-(4-METHYL-PIPERAZINO-METHYL)-BENZOYLAMIDO|-2-METHYLPHENYL -4-(3-PYRIDYL)-2PYRIMIDINE-AMINE AND A BIPHOSPHONATE**
KOMBINATION VON N- 5- 4-(4-METHYL-PIPERAZINO-METHYL)-BENZOYLAMIDO -2-METHYLPHENYL -4-(3-PYRIDIL)-2PYRIMIDINE-AMIN MIT EINEM BIPHOSPHONAT
COMBINAISON COMPRENANT N- 5- 4-(4-METHYL-PIPERAZINO-METHYL)-BENZOYLAMIDO|-2-METHYLPHENYL -4-(3-PYRIDYL)-2PYRIMIDINE-AMINE ET BIPHOSPHONATE

(30) Priority: 16.05.2001 US 291427 P
(43) Date of publication of application: 03.03.2004
(60) Divisional application: 06011049.1
(73) Proprietor: Novartis AG, 4056 Basel (CH); Novartis Pharma GmbH, 1230 Wien (AT)
(72) Inventor: BRUNS, Christian, 79110 Freiburg (DE); BUCHDUNGER, Elisabeth, 79395 Neuenburg (DE); O'REILLY, Terence, CH-4057 Basel (CH); SILBERMAN, Sandra, Leta, Randolph, NJ 07869 (US); WARTMANN, Markus, CH-4125 Riehen (CH); WECKBECKER, Gisbert, CH-4105 Biel-Benken (CH)
(74) Representative: de Weerd, Petrus G.W.
(86) International application number: PCT/EP2002/005362
(87) International publication number: WO 2002/092091

(56) References cited:
- WO-A-02/00024
- WO-A-02/28409
- WO-A-99/03854
- WO-A-02/067941
- FANG GUOFU ET AL: "CGP57148B (STI-571) induces differentiation and apoptosis and sensitizes Bcr-Abl-positive human leukemia cells to apoptosis due to antileukemic drugs." BLOOD, vol. 96, no. 6, 15 September 2000 (2000-09-15), pages 2246-2253, XP002217133 ISSN: 0006-4971
- TYLER THIESING J ET AL: "EFFICACY OF STI571, AN ABL TYROSINE KINASE INHIBITOR, IN CONJUNCTION WITH OTHER ANTILEUKEMIC AGENTS AGAINST BCR-ABL-POSITIVECELLS" BLOOD, W.B.SAUNDERS COMPAGNY, ORLANDO, FL, US, vol. 96, no. 9, 1 November 2000 (2000-11-01), pages 3195-3199, XP001035245 ISSN: 0006-4971
- KANO Y ET AL: "IN VITRO CYTOTOXIC EFFECTS OF A TYROSINE KINASE INHIBITOR STI571 IN COMBINATION WITH COMMONLY USED ANTILEUKEMIC AGENTS" BLOOD, W.B.SAUNDERS COMPAGNY, ORLANDO, FL, US, vol. 97, no. 7, 1 April 2001 (2001-04-01), pages 1999-2007, XP001035243 ISSN: 0006-4971
- TOPALY J ET AL: "SYNTERGISTIC ACTIVITY OF THE NEW ABL-SPECIFIC TYROSINE KINASE INHIBITOR ST1571 AND CHEMOTHERAPEUTIC DRUGS ON BCR-ABL-POSITIVE CHRONIC MYELOGENOUS LEUKEMIA CELLS" LEUKEMIA, MACMILLAN PRESS LTD, US, vol. 15, May 2001 (2001-05), pages 342-347, XP001039741 ISSN: 0887-6924

## Description

The invention relates to a combination comprising (a) N-{5-[4-(4-methyl-piperazino-methyl)-benzoylamido]-2-methylphenyl}-4-(3-pyridyl)-2-pyrimidine-amine and (b) a chemotherapeutic agent selected from the group consisting of biphosphonates and optionally at least one pharmaceutically acceptable carrier for simultaneous, separate or sequential use, in particular for the delay of progression or treatment of a proliferative disease, especially a solid tumor disease; a pharmaceutical composition comprising such a combination; the use of such a combination for the preparation of a medicament for the delay of progression or treatment of a proliferative disease; and to a commercial package or product comprising such a combination.

WO 02/00024 discloses the combination of lmatinib or its monomesylate salt and leptomycin B for the treatment of CML. WO 02/28409 discloses the combination of Imatinib or its monomesylate salt and farnesyl transferase inhibitors for the treatment of CML. WO02/067941 discloses the combination, a pharmaceutical combination or a kit of Imatinib or its monomesylate salt, an EGF-inhibitor and an epothilone derivative for the delay of progression or treatment of a proliferative disease. WO 99/03854 discloses the combination of Imatinib or its monomesylate salt with other antitumor agents for the treatment of a proliferative disease or transplant rejection. Kano et al, Blood, vol. 97 (7), April 2001, pages 1999-2007, describe the *in vitro* cytotoxic effects of Imatinib in combination with commonly used antileukemic agents.

Surprisingly, it has been found that the antineoplastic effect, i.e. especially the delay of progression or treatment of a proliferative disease, in particular the treatment of a tumor that is refractory to other chemotherapeutics known as antineoplastic agents, and/or the effect in treating acute or chronic transplant rejection, of a combination as defined herein is greater than the effects that can be achieved with either type of combination partner alone, i.e. greater than the effects of a monotherapy using only one of the combination partners (a) and (b) as defined herein.

Hence, the present invention pertains to a combination, such as a combined preparation or a pharmaceutical composition, which comprises (a) N-{5-[4-(4-methyl-piperazino-methyl)-benzoylamido]-2-methylphenyl}-4-(3-pyridyl)-2-pyrimidine-amine and (b) a chemotherapeutic agent selected from the group consisting of biphosphonates, wherein the active ingredients are present in each case in free form or in the form of a pharmaceutically acceptable salt, and optionally at least one pharmaceutically acceptable carrier; for simultaneous, separate or sequential use.

The term "a combined preparation", as used herein defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b). The ratio of the total amounts of the combination partner (a) to the combination partner (b) to be administered in the combined preparation can be varied, e.g. in order to cope with the needs of a patient subpopulation to be treated or the needs of the single patient which different needs can be due to the particular disease, age, sex, body weight, etc. of the patients. Preferably, there is at least one beneficial effect, e.g., a mutual enhancing of the effect of the combination partners (a) and (b), in particular a synergism, e.g. a more than additive effect, additional advantageous effects, less side effects, a combined therapeutical effect in a non-effective dosage of one or both of the combination partners (a) and (b), and very preferably a strong synergism of the combination partners (a) and (b).

The term "delay of progression" as used herein means administration of the combination to patients being in a pre-stage or in an early phase of the disease to be treated, in which patients for example a pre-form of the corresponding disease is diagnosed or which patients are in a condition, e.g. during a medical treatment or a condition resulting from an accident, under which it is likely that a corresponding disease will develop.

The term "solid tumor" especially means breast cancer, cancer of the colon and generally the Gl tract, lung cancer, in particular small-cell lung cancer, and non-small-cell lung cancer, head and neck cancer, genitourinary cancer, e.g. cervical, uterine, ovarian, testicles, prostate or bladder cancer; Hodgkin's disease or Kaposi's sarcoma. The combinations of the present invention inhibit the growth of liquid tumors and, in particular, solid tumors. Furthermore, depending on the tumor type and the particular combination used a decrease of the tumor volume can be obtained. The combinations disclosed herein are also suited to prevent the metastatic spread of tumors and the growth or development of micrometastases. The combinations disclosed herein are in particular suitable for the treatment of poor prognosis patients, e.g. such poor prognosis patients having non-small-cell lung cancer.

It will be understood that references to the combination partners (a) and (b) are meant to also include the pharmaceutically acceptable salts. If these combination partners (a) and (b) have, for example, at least one basic center, they can form acid addition salts. Corresponding acid addition salts can also be formed having, if desired, an additionally present basic center. The combination partners (a) and (b) having an acid group (for example COOH) can also form salts with bases. The combination partner (a) or (b) or a pharmaceutically acceptable salt thereof may also be used in form of a hydrate or include other solvents used for crystallization. N-{5-[4-(4-methyl-piperazino-methyl)-benzoylamido]-2-methylphenyl}-4-(3-pyridyl)-2-pyrimidine-amine, i.e. combination partner (a), is preferably used in the present invention in the form of its monomesylate salt (STI571).

The combination partner (a) can be prepared and administered as described in WO 99/03854, especially the monomesylate salt of N-{5-[4-(4-methyl-piperazino-methyl)-benzoylamido]-2-methylphenyl}-4-(3-pyridyl)-2-pyrimidine-amine can be formulated as described in Examples 4 and 6 of WO 99/03854.

The term "bisphosphonates" as used herein includes, but is not limited to etridonic acid, clodronic acid, tiludronic acid, pamidronic acid, alendronic acid, ibandronic acid, risedronic acid and zoledronic acid. "Etridonic acid" can be administered, e.g., in the form as it is marketed, e.g. under the trademark DIDRONEL^{™}. "Clodronic acid" can be administered, e.g., in the form as it is marketed, e.g. under the trademark BONEFOS^{™}. "Tiludronic acid" can be administered, e.g., in the form as it is marketed, e.g. under the trademark SKELID^{™}. "Pamidronic acid" can be administered, e.g., in the form as it is marketed, e.g. under the trademark AREDIA^{™}. "Alendronic acid" can be administered, e.g., in the form as it is marketed, e.g. under the trademark FOSAMAX^{™}. "Ibandronic acid" can be administered, e.g., in the form as it is marketed, e.g. under the trademark BONDRANAT^{™}. "Risedronic acid" can be administered, e.g., in the form as it is marketed, e.g. under the trademark ACTONEL^{™}. "Zoledronic acid" can be administered, e.g., in the form as it is marketed, e.g. under the trademark ZOMETA^{™}. Zoledronic acid has been specifically described in the European patent No. 0 275 821, published on July 27, 1988, as well as in US patent No. 4,939,130 granted on July 03, 1990, and Japanese Patent No. 2744238 all in the name of the applicant. The term "Zoledronic acid" also includes pharmacologically acceptable salt thereof or any hydrate thereof such as anhydrous zoledronic acid, zoledronic acid monohydrate, disodium zeldronate, trisodium zeldronate.

The structure of the active agents identified by code nos., generic or trade names may be taken from the actual edition of the standard compendium "The Merck Index" or from databases, e.g. Patents International (e.g. IMS World Publications).

A combination which comprises (a) N-{5-[4-(4-methyl-piperazino-methyl)-benzoylamido}-2-methylphenyl}-4-(3-pyridyl)-2-pyrimidine-amine and (b) a chemotherapeutic agent selected from the group consisting of biphosphonates,, in which the active ingredients are present in each case in free form or in the form of a pharmaceutically acceptable salt and optionally at least one pharmaceutically acceptable carrier, will be referred to hereinafter as a COMBINATION OF THE INVENTION.

The nature of proliferative diseases like solid tumor diseases and acute or chronic transplant rejection is multifactorial. Under certain circumstances, drugs with different mechanisms of action may be combined. However, just considering any combination of drugs having different mode of action does not necessarily lead to combinations with advantageous effects.

All the more surprising is the experimental finding that *in vivo* the administration of a COMBINATION OF THE INVENTION results not only in a beneficial effect, especially a synergistic therapeutic effect, e.g. with regard to slowing down, arresting or reversing the neoplasm formation or a longer duration of tumor response, but also in further surprising beneficial effects, e.g. less side-effects, an improved quality of life and a decreased mortality and morbidity, compared to a monotherapy applying only one of the pharmaceutically active ingredients used in the COMBINATION OF THE INVENTION, in particular in the treatment of a tumor that is refractory to other chemotherapeutics known as anti-cancer agents. In particular, an increased up-take of the combination partner (b) in tumor tissue and tumor cells is observed, when applied in combination with combination partner (a).

A further benefit is that lower doses of the active ingredients of the COMBINATION OF THE INVENTION can be used, for example, that the dosages need not only often be smaller but are also applied less frequently, or can be used in order to diminish the incidence of side-effects. This is in accordance with the desires and requirements of the patients to be treated.

It can be shown by established test models and in particular those test models described herein that a COMBINATION OF THE INVENTION results in a more effective delay of progression or treatment of a proliferative disease and/or acute or chronic transplant rejection compared to the effects observed with the single combination partners. The person skilled in the pertinent art is fully enabled to select a relevant test model to prove the hereinbefore and hereinafter mentioned therapeutic indications and beneficial effects. The pharmacological activity of a COMBINATION OF THE INVENTION may, for example, be demonstrated in a clinical study or in a test procedure as essentially described hereinafter.

Suitable clinical studies are, for example, open label non-randomized, dose escalation studies in patients with advanced solid tumors. Such studies prove in particular the synergism of the active ingredients of the COMBINATIONS OF THE INVENTION. The beneficial effects on proliferative diseases can be determined directly through the results of these studies or by changes in the study design which are known as such to a person skilled in the art. Such studies are, in particular, suitable to compare the effects of a monotherapy using the active ingredients and a COMBINATION OF THE INVENTION. Preferably, the combination partner (a) is administered with a fixed dose and the dose of the combination partner (b) is escalated until the Maximum Tolerated Dosage is reached. In a preferred embodiment of the study, each patient receives daily doses of the combination partner (a). The efficacy of the treatment can be determined in such studies, e.g., after 18 or 24 weeks by radiologic evaluation of the tumors every 6 weeks.

Alternatively, a placebo-controlled, double blind study can be used in order to prove the benefits of the COMBINATION OF THE INVENTION mentioned herein.

The COMBINATION OF THE INVENTION can also be applied in combination with surgical intervention, mild prolonged whole body hyperthermia and/or irradiation therapy.

In a preferred embodiment of the invention the bisphosphonate is especially selected from pamidronic acid and zoledronic acid.

COMBINATION OF THE INVENTION is also useful for preventing or combating graft vessel diseases, e.g. allo- or xenotransplant vasculopathies, e.g. graft vessel atherosclerosis or chronic graft rejection, e.g. in a transplant of organ, tissue or cells, e.g. heart, lung, combined heart-lung, liver, kidney or pancreatic transplants (e.g. pancreatic islet cells), or for preventing or treating vein graft stenosis, restenosis and/or vascular occlusion following vascular injury, e.g. caused by catherization procedures or vascular scraping procedures such as percutaneous transluminal angioplasty, laser treatment or other invasive procedures which disrupt the integrity of the vascular intima or endothelium.

The COMBINATION OF THE INVENTION can be a combined preparation or a pharmaceutical composition.

It is one objective of this invention to provide a pharmaceutical composition comprising a quantity, which is jointly therapeutically effective against a proliferative disease or acute or chronic transplant rejection comprising the COMBINATION OF THE INVENTION. In this composition, the combination partners (a) and (b) can be administered together, one after the other or separately in one combined unit dosage form or in two separate unit dosage forms. The unit dosage form may also be a fixed combination.

The pharmaceutical compositions for separate administration of the combination partners (a) and (b) and for the administration in a fixed combination, i.e. a single galenical compositions comprising at least two combination partners (a) and (b), according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

Novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partner of the COMBINATION OF THE INVENTION may be administered simultaneously or sequentially and in any order, and the components may be administered separately or as a fixed combination. For example, the method of delay of progression or treatment of a proliferative disease and/or acute or chronic transplant rejection according to the invention may comprise (i) administration of the combination partner (a) in free or pharmaceutically acceptable salt form and (ii) adminstration of a combination partner (b) in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimes of simultaneous or alternating treatment and the term "administering" is to be interpreted accordingly.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites.

N-{5-[4-(4-methyl-piperazino-methyl)-benzoylamido]-2-methylphenyl}-4-(3-pyridyl)-2-pyrimidine-amine monomesylate, is preferably administered to a human in a dosage in the range of about 2.5 to 850 mg/day, more preferably 5 to 600 mg/day and most preferably 20 to 300 mg/day. Unless stated otherwise herein, the compound is preferably administered from one to four times per day.

Single doses of Zoledronic acid should preferably not exceed 8 mg; preferably 4 mg more preferably from about 0.5 to about 4 mg. The duration of an intravenous infusion should preferably be no less than 15 minutes. Compositions containing zoledronic acid are described in the art, e.g. in the International patent application US01/14886 filed on May 5, 2001, and published on November 29, 2001.

Alendronic acid may be administered to a human in a dosage range varying from about 5 to 10 mg/day.

Clodronic acid may be administered to a human e.g. in a dosage range varying from about 750 to 1500 mg/day.

Etridonic acid may be administered to a human in a dosage range varying from about 200 to 400 mg/day.

Ibandronic acid may be administered to a human in a dosage range varying from about 1 to 4 mg every three to four weeks.

Risedronic acid may be administered to a human in a dosage range varying from about 20 to 30 mg/day.

Pamidronic acid may be administered to a human in a dosage range varying from about 15 to 90 mg every three to four weeks.

Tiludronic acid may be administered to a human in a dosage range varying from about 200 to 400 mg/day.

Furthermore, the present invention pertains to the use of a COMBINATION OF THE INVENTION for the delay of progression or treatment of a proliferative disease and/or acute or chronic transplant rejection and for the preparation of a medicament for the delay of progression or treatment of a proliferative disease and/or acute or chronic transplant rejection.
Moreover, the present invention provides a commercial package comprising as active ingredients COMBINATION OF THE INVENTION, together with instructions for simultaneous, separate or sequential use thereof in the delay of progression or treatment of a proliferative disease and/or acute or chronic transplant rejection.

The following Example illustrates the invention described above. Other test models known as such to the person skilled in the pertinent art can also determine the beneficial effects of the COMBINATION OF THE INVENTION.

### Example 1: DU145 prostate carcinoma human cell lines grown i.d. in nude mice

DU145 prostate carcinoma human cell lines are grown i.d. in nude mice. Tumor cell (10⁶) are injected intradermally (i.d.) on the left and right flank of nude mice. Treatment with compounds is started after 25-32 days when tumors reach a size of 80-100 mm². At this time animals are sorted into groups with equivalent mean and range of tumor sizes. Treatment is then randomized to the different groups. Tumor size is measured with calipers on a weekly basis.

Example 2 (Reference Example): STI571 alone, Taxol^{®} alone (paclitaxel), doxorubicin alone or the combination of STI571 plus one of both versus rat C6 glioma tumor xenografts in female BALB/c mice

Tumors are initiated by the s.c. injection of 1 x 10⁶ rat C6 cells (n=8/group). When the tumors reaches ~ 75 mm³, ST1571 treatment is begun at 100 mg/kg, p.o., every 12h. The combination partner Taxol^{®} is administered at 15 mg/kg, i.v., every 48 hours, for five times, or the combination partner doxorubicin at 9 mg/kg, i.v., every 7 days.
Data points just spanning the IC50 of the agents alone or in combination are entered into the CalcuSyn program (CalcuSyn, Biosoft, Cambridge UK). This program calculates a non-exclusive combination index (Cl), whose value is indicative of the interaction of the two compounds, where Cl ~ 1 represents nearly additive effects; 0.85 - 0.9 indicates a slight synergism and a value below 0.85 indicates synergy.
A C.I. of 0.3 ± 0.03 was obtained for the combination of STI571 and Taxol^{®} and a C.I. of 0.4 ± 0.04 for the combination of STI571 and doxorubicin.

In an *in vitro* C6 glioma test a C.I. of 0.3 ± 0.2 for the combination of STI571 and vinblastine was observed.

The same experimental protocol can be used to prove that STI571 induces synergistic interactions with other drugs as mentioned above.
The surprising beneficial effect of our new combinations, e.g. less side effects, synergistic therapeutic effect, can also be demonstrated in clinical studies as essentially described hereinafter in a manner known to the skilled person.

### Example 3:

The purpose of this study is to determine the effects of treatment of patients with osteolytic or osteoblastic bone metastases with the combination of Glivec + Zometa.

### STUDY OJECTIVES:

The primary objective of this study is to prove that there are synergistic drug interactions and no side-effects between Glivec and Zometa (pharmacokinetic, pharmacologic, toxic).
The secondary objective is to determine time to disease progression (skeletal, extra-skeletal) for patients treated with Glivec + Zometa.
Other objectives are to determine pain scores, analgesic scores, skeletal-related events, objective bone lesion responses, bone resorption markers, and overall survival for patients treated with Glivec + Zometa. It is also an objective to evaluate the efficacy of Glivec + Zometa in palliative treatment of painful bony metastases.

### DURATION OF STUDY:

Time permitted for patient enrollment: 12 months
Duration of an individual patient's participation: 12 months
Total duration of study: 24 months

### STUDY POPULATION: 15 Patients

Inclusion Criteria: Any cancer with at least one bone metastasis (lytic or blastic) on plain film (≥ 1 cm). Patients may be receiving anti-cancer therapy at the time of randomization. Ambulatory patients aged ≥18 years or the age of majority.
ECOG (Eastern Cooperative Oncology Group) performance status of 0, 1 or 2.

If the patient is of childbearing potential, a negative pregnancy test is required prior to treatment. Ability to comply with trial requirements.

### Interruption or Discontinuation of Treatment:

It is documented whether or not each patient completed the clinical study. If for any patient either study treatment or observations were discontinued the reason is recorded. Patients discontinuing trial treatment before 12 months are not replaced. Reasons that a patient may discontinue participation in a clinical study are considered to constitute one of the following: Adverse event(s), abnormal laboratory value(s), abnormal test procedure result(s), unsatisfactory therapeutic effect, subject's condition no longer requires study treatment, protocol violation, subject withdrew consent, lost to follow-up, administrative problems, death.

### TREATMENT:

All patients receive the following, A) Zometa 4 mg as a 15 minute IV infusion every 3-4 weeks + Glivec 400, 600 or 800 mg P.O. daily (The 800 mg dose of Glivec is given as 400 mg bid.). On study day 1, eligible patients receive a 4 mg/15 min infusion of Zometa with attendant pharmacokinetic sampling. B) Glivec p.o. dosing is initiated on study day 3. Pharmacokinetic sampling for steady-state imatinib concentrations takes place over an 8-hour period on any day during days 10-14, and on day 28 (coincides with second dose of Zometa). Study drug supplies (Zometa and Glivec) will be shipped to the pharmacist at each center. Study drugs will be supplied for the 12-month study period. Drugs are packaged in an open-label fashion.
Investigational therapy: Zometa is supplied in 4 mg lyophilized vials. Each 4 mg vial is to be reconstituted with 5 ml of sterile water. The appropriate volume of reconstituted Zometa is to be mixed with normal (0.9%) saline so that the total volume infused is 100 ml. Reconstituted Zometa is to be administered as a 15-minute i.v. infusion.
If reconstituted Zometa solutions cannot be used immediately, solutions must be refrigerated at temperatures between 36-46°F (2-8°C) and can be used for up to 24 hours.

Three patients are treated at 400, 600 or 800 mg per day with Glivec. If there are grade 3 or 4 toxicities in any patients, the number of patients at that dose of Glivec are expanded to 6 patients. The MTD (maximum tolerated dose) of Glivec is reached if 2/6 patients exhibit grade 3 or 4 toxicity. (See NCI/NIH Common Toxicity Criteria)

if an individual patient experiences grade 3 or 4 toxicity, treatment under this study with Zometa + Glivec is discontinued. That patient then receives treatment as directed by his/her physician.
An individual patient may continue to receive Zometa + Glivec for up to 12 months if there is disease progression in bone.

Standard Anti-Cancer Therapy: Patients must be receiving anti-cancer therapy at the time of randomization. Breast cancer patients receiving hormonal therapy must be on first-line or second-line hormonal therapy for metastatic disease. Breast cancer patients must not be receiving greater than second-line hormonal therapy for metastatic disease at the time of randomization, unless being given in combination with chemotherapy. Changes to the initial anti-neoplastic regimen during the study are at the discretion of the treating physician. Patients in both disease groups may discontinue all specific anti-neoplastic therapies at any time during the trial. Patients who are no longer receiving anti-neoplastic therapies must continue to receive study drug infusions at 3-4 weekly intervals until the stipulated 12-months of therapy is completed.

Blood Chemistry: During ongoing monitoring of the phase III trials with Zometa® the safety monitoring boards noted a possibly higher frequency of increases in serum creatinine and AEs associated with the use of Zometa® and recommended that serum creatinine is measured prior to each dose of study drug. A 72-hour window for checking creatinine is allowed prior to the next dose. The local serum creatinine result is evaluated according to the following criteria:
If the patient's baseline serum creatinine was < 1.4 mg/dl at the time of study entry, an increase of 0.5 mg/dl or more requires the delaying of the dose of study drug until the patient's serum creatinine returns to no higher than 10% above the baseline value.
If the patient's baseline serum creatinine was ≥ 1.4 mg/dl, then any increase in the serum creatinine of 1.0 mg/dl or more requires that the study drug be delayed until the patients serum creatinine returns to no higher than 10% above the baseline value.
Any doubling of the baseline serum creatinine value requires that the study drug be delayed until the patient's serum creatinine returns to no higher than 10% above the baseline value. Should the study drug need to be delayed, the patient's serum creatinine continues to be followed at intervals according to the Investigator's clinical judgment, but at least at the regularly scheduled study visits until full recovery (i.e., return to no higher than 10% above the baseline value). Other study procedures should be followed according to the protocol schedule even if study drug continues to be held.
If the patient experiences a Grade 3/4 hematological toxicity, defined as an ANC < 1 x 10⁹/L, or a platelet count < 50 x 10⁹/L, Glivec must be withheld until the toxicity has resolved to ≤ Grade 2. ANC (absolute neutrophil count) will take precedence over a WBC (white blood cell) count in determining the degree of neutropenia (e.g. doses should not be interrupted for a patient with a WBC < 2.0 x 10⁹ /L but ANC > 1 x 10⁹/L). If the toxicity resolves to Grade 1 within two weeks, Glivec treatment may be resumed at the same dose. If the Grade 3/4 toxicity recurs or persists for longer than two weeks, Glivec must be withheld and then recommended at the daily dose reduced by 200 mg (e.g. from 800 mg/day to 600 mg/day) once toxicity has resolved to ≤ Grade 1. If the Grade 3/4 toxicity recurs again, Glivec must be stopped. No dose reductions will be performed again for grade 1-4 anemia. If the patient develops anemia, s/he may be transfused at the discretion of the investigator.
The optimal dose of Glivec in an as-yet undefined spectrum of diseases is unknown, however, the possibility of a clinically relevant dose-response may be of importance. Therefore, it is possible that higher doses of Glivec might induce responses even if a lower dose fails. If vomiting occurs, no additional trial medication should be taken that day in an effort to replace the material that has been vomited.
If for any reason, interruption of treatment with Glivec is equal or longer than 14 days, than the patient should be discontinued.

Skeletal-Related Events: SREs are defined as pathologic bone fractures, spinal cord compression, surgery to bone and radiation therapy to bone.
Pathologic fractures are defined as bone fractures which occur spontaneously or which result from trivial trauma. A new vertebral compression fracture is defined as a decrease in total vertebral height, or anterior vertebral height, or posterior vertebral height of ≥ 25% from baseline (Visit 1). An old (pre-existing) vertebral compression fracture may be present at Visit 1. At visit 1, a pre-existing vertebral compression fracture is defined as a decrease in total, or anterior or posterior vertebral height of ≥ 25% as compared to a previous spinal film or as compared to an adjacent vertebrae. A further reduction in the total, or anterior, or posterior vertebral height of an old vertebral fracture by ≥ 25% during the study is to be classified as a new vertebral compression fracture. Each pathologic fracture (vertebral and non-vertebral, including rib fractures) is to be documented by a plain X-ray film during the study and is to be counted separately.

Overall Progression of Disease: Overall progression of disease is determined every 3 months by the investigator using the tumor response criteria outlined in a form A (for multiple myeloma patients) and B (for breast cancer patients). The assessment of tumor progression must take into account the radiologist's assessment of serial bone radiographic studies and appropriate serial radiographic studies of non-skeletal tumor sites, if present.
ECOG Performance Status: ECOG performance status is completed at least every 3 months.
Quality of Life (QOL): QOL is assessed by using a FACT-G (Functional Assessment of Cancer Therapy General) questionnaire every 3 months. The quality of life questionnaires is completed by the patient upon arrival at the clinic and before the patient has either been interviewed by the physician or received study medication.
Analgesic Scores: Analgesic use is scored every 3 months according to the analgesic score. Pain Score: The Pain Score is assessed every 3 months according to the brief Pain Inventory (BPI) short form. The BPI can produce three pain scores: worst pain, a composite pain score and a pain interference score. The composite pain score is used in this study. Markers of Bone Resorption: The following parameters are be measured by a central laboratory:
- Serum PTH (parathyroid hormone) (Visits 1, 6, 10, 14 and 19). Measurement of PTH using an immuno-chemiluminescent assay (ICMA-PTH) is performed. Draw a sample (5ml) in a 5.0 ml red-top or SST (serum separator) tube and centrifuge. Immediately after centrifugation, remove serum (2 ml) and place in plastic transport vial and freeze. Send the frozen serum in plastic vials on dry ice to the central laboratory. The data remain blinded to sites.
- BAP (every 3 months). Venous blood will be drawn for BAP (bone alkaline phosphatase). The data remain blinded to the study monitors and investigator sites.
- Urine chemistry (every 3months). After rising in the morning, the initial urine sample passed by the patient is discarded. The subsequent 'second void' urine is collected. The patient need not remain fasting but to reduce diurnal variation other sample is collected in mid-morning whenever possible. Urine must be mixed thoroughly before taking aliquots for creatinine, pyridinoline, deoxypridinoline and N-telopeptide. The data remain blinded to the study monitors and investigator sites.
Safety Assessments: Safety assessments consist of monitoring and recording all adverse event and serious adverse events, the regular monitoring of hematology, blood chemistry and urine values, and the performance of physical examinations. In addition the time to discontinuation of study drug is evaluated and survival will be assessed.
Drug levels and pharmacokinetic assessments: A blood sample (5mL) for the determination of serum concentration of alpha-1 acid glycoprotein is obtained at visit 1.
Sequential blood samples (5 mL) or the determination of plasma concentrations of zoledronic acid are obtained at visit 2 as follows; 0h (pre start infusion), 15 min (within 30 seconds post end infusion), 45 min, 1.5h, 3h, 5h, 8h.
Sequential bloods samples (5 mL, 7.5 mL for 0h sample) for the determination of plasma concentrations of imatinib (imatinib and zoledronic acid in 0-h sample) are obtained at visit 2A (any time between days 10-14 post first dose Zometa) as follows: 0h (pre-oral dose), 1.5 h, 3h, 5h, 8h.
Sequential blood samples (7.5 mL) for the determination of plasma concentrations of imatinib and zoledronic acid are obtained at visit 3 as follows: 0h (pre-oral Glivec and i.v. Zometa), 15 min, 45 min, 1.5h, 3h, 5h, 8h.
Sequential blood samples (7.5 mL) for the determination of plasma concentrations of imatinib and zoledronic acid are obtained at visit 6 as follows: 0h (pre-oral Glivec and i.v. Zometa), 15 min, 45 min, 1.5h, 3h, 5h, 8h.
These samples are stored at -20C and shipped to a laboratory to measure Glivec (imatinib) and Zometa (zoledronic acid) levels.

By this study it is shown that the toxicity profile of the Glivec/Zometa combination is superior to that of other combinations. Furthermore, the Glivec/Zometa combination shows a synergistic therapeutic effect, higher efficacy, efficacy in palliative treatment of painful bony metastases, and other beneficial effects as mentioned in the above description.

### Example 4:

### PROTOCOL SUMMARY

Study Objectives: To prove the safety and efficacy of Glivec (imatinib mesylate, ST1571) and Zometa (zoledronic acid) in the palliative treatment of painful bony metastases due to androgen-independent prostate cancer. Prove that the Glivec/Zometa combination shows a synergistic therapeutic effect, higher efficacy, improved safety and other beneficial effects as mentioned in the above description.
Patient Inclusion Criteria: Patients with histologically confirmed prostate cancer who have progressed on standard hormonal management (including antiandrogen withdrawal) and have symptomatic bone metastases are eligible. Eligible patients continue primary hormonal therapy, have a PSA (prostate specific antigen) of ≥ 5 ng/ml, an ECOG performance status ≤ 3, and adequate hematologic, hepatic and renal function.
Patient Exclusion Criteria: Patients with serious medical illness, active second malignancy other than non-melanoma skin cancers will not be eligible. Patients who have received radiotherapy within 30 days, bisphosphonates within 6 months, radiopharmaceuticals within 60 days, or investigational therapy within 30 days will be excluded from the study.

Treatment Schedule: Zometa 4 mg i.v. every 28 days and Glivec 600 mg po q.d.

Primary Objective: Pain response defined as a 2-point reduction on the 6 point present pain intensity scale of the mMcGill-Melzack Pain Questionnaire (Tannock I. et al.. Treatment of metastatic prostatic cancer with low-dose prednisone: evaluation of pain and quality of life as pragmatic indices of response. *J Clin Oncol.* 1989;7(5):590-7; Melzack R. The McGill Pain Questionnaire: major properties and scoring methods. *Pain.* 1975;1:277-299) maintained on 2 consecutive evaluations at least 4 weeks apart without an increase in analgesic consumption.

Secondary Objectives: 50% decrease in analgesic medication use without an increase in pain maintained on 2 consecutive evaluations at least 4 weeks apart.
PSA (prostate specific antigen) response defined as a 50% decrease in PSA maintained on 2 consecutive evaluations at least 4 weeks apart.
Impact of therapy on markers of bone turnover, specifically: serum bone specific alkaline phosphatase, serum osteocalcin, and urine N-telopeptides prior to treatment, and after 2, 4, 8, and 12 weeks of treatment.
Time to progression measured by PSA and pain endpoints.
Impact on quality of life measured by the EORTC core questionnaire QLQ-C30 (Aaranson NK, et al. The European Organization for Research and Treatment of Cancer QLQ-C30: a quality-of-life instrument for use in international clinical trials in oncology. *Journal of the National Cancer Institute.* 1993;85(5):365-376; Osoba D et al. Psychometric properties and responsiveness of the EORTC Quality of Life Questionnaire (QLQ-C30) in patients with breast, ovarian, and lung cancer. *Quality of Life Research.* 1994;3(353-364)) and a module specific for prostate cancer designed according to EORTC guidelines. Toxicity of the GLIVEC/Zometa combination.

## Claims

1. A combination which comprises (a) N-{5-[4-(4-methyl-piperazino-methyl)-benzoylamido]-2-methylphenyl}-4-(3-pyridyl)-2-pyrimidine-amine and (b) a chemotherapeutic agent selected from the group consisting of biphosphonates wherein the active ingredients are present in each case in free form or in the form of a pharmaceutically acceptable salt or any hydrate thereof.

2. The combination according to claim 1 wherein the compound (a) is used in the form of its monomethanesulfonate salt.

3. The combination according to claim 1 or 2 which is a combined preparation or a pharmaceutical composition.

4. The combination according to any one of claims 1 to 3 wherein the chemotherapeutic agent (b) is zoledronic acid.

5. The combination according to anyone of claims 1 to 4 for simultaneous, separate or sequential use.

6. The combination according to anyone of claims 1 to 5 which is a fixed combination.

7. The combination according to any one of claims 1 to 6 for use in the treatment of human or animal body.

8. Use of a combination according to any one of claims 1 to 7 for the preparation of a medicament for the delay of progression or treatment of a proliferative disease.

9. Use of a combination according to any one of claims 1 to 7 for the preparation of a medicament for the delay of progression or treatment of acute or chronic transplant rejection.

10. Use of a combination according to any one of claims 1 to 7 for the preparation of a medicament for the palliative treatment of painful bony metastases.

11. A pharmaceutical composition comprising a combination according to any one of claims 1 to 7 in a quantity which is jointly therapeutically effective against a proliferative disease or acute or chronic transplant rejection and at least one pharmaceutically acceptable carrier.

12. A commercial package comprising (a) N-{5-[4-(4-methyl-piperazino-methyl)-benzoyl-amido]-2-methylphenyl}-4-(3-pyridyl)-2-pyrimidine-amine and (b) a chemotherapeutic agent selected from biphosphonates wherein the active ingredients are present in each case in free form or in the form of a pharmaceutically acceptable salt or any hydrate thereof, together with instructions for simultaneous, separate or sequential use thereof.

13. A commercial package according to claim 12 wherein the chemotherapeutic agent (b) is zoledronic acid.

## Patentansprüche

1. Kombination, umfassend (a) N-{5-[4-(4-Methylpiperazinomethyl)benzoylamido]-2-methylphenyl}-4-(3-pyridyl)-2-pyrimidinamin und (b) ein Chemotherapeutikum, das aus der Gruppe ausgewählt ist, die besteht aus Biphosphonaten, worin die Wirkstoffe jeweils in freier Form oder in Form eines pharmazeutisch akzeptablen Salzes oder eines Hydrats hiervon vorhanden sind.

2. Kombination nach Anspruch 1, worin die Verbindung (a) in Form ihres Monomethansulfonatsalzes verwendet wird.

3. Kombination nach Anspruch 1 oder 2, die ein Kombinationspräparat oder eine pharmazeutische Zusammensetzung ist.

4. Kombination nach einem der Ansprüche 1 bis 3, worin das Chemotherapeutikum (b) Zoledronsäure ist.

5. Kombination nach einem der Ansprüche 1 bis 4 zur simultanen, separaten oder sequentiellen Anwendung.

6. Kombination nach einem der Ansprüche 1 bis 5, die eine fixe Kombination ist.

7. Kombination nach einem der Ansprüche 1 bis 6 zur Verwendung für die Behandlung eines menschlichen oder tierischen Körpers.

8. Verwendung einer Kombination nach einem der Ansprüche 1 bis 7 zur Herstellung eines Arzneimittels zur Verzögerung einer Progression oder zur Behandlung einer proliferativen Krankheit.

9. Verwendung einer Kombination nach einem der Ansprüche 1 bis 7 zur Herstellung eines Arzneimittels für die Verzögerung einer Progression oder die Behandlung einer akuten oder chronischen Transplantatabstoßung.

10. Verwendung einer Kombination nach einem der Ansprüche 1 bis 7 zur Herstellung eines Arzneimittels für die palliative Behandlung schmerzvoller Knochenmetastasen.

11. Pharmazeutische Zusammensetzung, umfassend eine Kombination nach einem der Ansprüche 1 bis 7 in einer Menge, die gemeinsam therapeutisch wirksam ist gegen eine proliferative Krankheit oder eine akute oder chronische Transplantatabstoßung, und wenigstens einen pharmazeutisch akzeptablen Träger.

12. Handelspackung, umfassend (a) N-{5-[4-(4-Methylpiperazinomethyl)-benzoylamido]-2-methylphenyl}-4-(3-pyridyl)-2-pyrimidinamin und (b) ein Chemotherapeutikum, das aus der Gruppe ausgewählt ist, die besteht aus Biphosphonaten, worin die Wirkstoffe jeweils in freier Form oder in Form eines pharmazeutisch akzeptablen Salzes oder eines Hydrats hiervon vorliegen, zusammen mit Instruktionen zur simultanen, separaten oder sequentiellen Anwendung hiervon.

13. Handelspackung nach Anspruch 12, worin das Chemotherapeutikum (b) Zoledronsäure ist.

## Revendications

1. Combinaison qui comprend (a) de la N-{5-[4-(4-méthyl-pipérazino-méthyl)-benzoylamido]-2-méthylphényl}-4-(3-pyridyl)-2-pyrimidine-amine et (b) un agent chimiothérapeutique choisi dans le groupe constitué par les biphosphonates, dans laquelle les principes actifs sont présents dans chaque cas sous forme libre ou sous la forme d'un sel pharmaceutiquement acceptable ou de tout hydrate de celui-ci.

2. Combinaison selon la revendication 1, dans laquelle le composé (a) est utilisé sous la forme de son sel monométhanesulfonate.

3. Combinaison selon la revendication 1 ou 2, qui est une préparation combinée ou une composition pharmaceutique.

4. Combinaison selon l'une quelconque des revendications 1 à 3, dans laquelle l'agent chimiothérapeutique (b) est de l'acide zolédronique.

5. Combinaison selon l'une quelconque des revendications 1 à 4 pour utilisation simultanée, séparée ou séquentiel.

6. Combinaison selon l'une quelconque des revendications 1 à 5, qui est une combinaison fixe.

7. Combinaison selon l'une quelconque des revendications 1 à 6 utilisée dans le traitement de l'organisme humain ou animal.

8. Utilisation d'une combinaison selon l'une quelconque des revendications 1 à 7 pour la préparation d'un médicament pour le ralentissement de la progression ou le traitement d'une maladie proliférative.

9. Utilisation d'une combinaison selon l'une quelconque des revendications 1 à 7 pour la préparation d'un médicament pour le ralentissement de la progression ou le traitement du rejet de greffe aigu ou chronique.

10. Utilisation d'une combinaison selon l'une quelconque des revendications 1 à 7 pour la préparation d'un médicament pour le traitement palliatif des métastases osseuses douloureuses.

11. Composition pharmaceutique comprenant une combinaison selon l'une quelconque des revendications 1 à 7 en une quantité qui est conjointement thérapeutiquement efficace contre une maladie proliférative ou un rejet de greffe aigu ou chronique et au moins un véhicule pharmaceutiquement acceptable.

12. Conditionnement commercial comprenant (a) de la N-{5-[4-(4-méthyl-pipérazino-méthyl)-benzoylamido]-2-méthylphényl}-4-(3-pyridyl)-2-pyrimidine-amine et (b) un agent chimiothérapeutique choisi parmi les biphosphonates, dans lequel les principes actifs sont présents dans chaque cas sous forme libre ou sous la forme d'un sel pharmaceutiquement acceptable ou de tout hydrate de celui-ci, conjointement avec le mode d'emploi pour leur utilisation simultanée, séparée ou séquentiel.

13. Conditionnement commercial selon la revendication 12 dans lequel l'agent chimiothérapeutique (b) est de l'acide zolédronique.
